(19) Europäisches Patentamt
European Patent Office
Office européen des brevets

(11) Veröffentlichungsnummer: **0 278 445**
**B1**

(12) **EUROPÄISCHE PATENTSCHRIFT**

(45) Veröffentlichungstag der Patentschrift: **30.01.91**

(21) Anmeldenummer: **88101764.4**

(22) Anmeldetag: **06.02.88**

(51) Int. Cl.$^5$: **C 07 D 231/12,** C 07 D 231/16, C 07 D 233/60, C 07 D 233/68, C 07 D 249/08, C 09 K 17/00

(54) **Azolhalbaminal-Derivate und ihre Verwendung als Nitrifikationsinhibitoren.**

(30) Priorität: **12.02.87 DE 3704359**

(43) Veröffentlichungstag der Anmeldung: **17.08.88 Patentblatt 88/33**

(45) Bekanntmachung des Hinweises auf die Patenterteilung: **30.01.91 Patentblatt 91/05**

(84) Benannte Vertragsstaaten: **AT BE CH DE ES FR GB GR IT LI NL SE**

(56) Entgegenhaltungen:
**CHEMICAL ABSTRACTS, Band 97, Nr. 21, 22. November 1982, Columbus, Ohio, USA; KOFMAN, T.P.; KIRPENKO, Z.V.; PEVZNER, M.S.: "Heterocyclic nitro compounds. 28. 1-(oxoalkyl)-3-nitro-5-substituted-1,2,4-triazoles in reactions with hydrazine and hydroxylamine" Seite 818, Spalte 2, Zusammenfassung-Nr. 182306n**

(73) Patentinhaber: **BASF Aktiengesellschaft**
**Carl-Bosch-Strasse 38**
**D-6700 Ludwigshafen (DE)**

(72) Erfinder: **Wagner, Klaus, Dr.**
**Sauterstrasse 32**
**D-6730 Neustadt (DE)**
Erfinder: **Rieber, Norbert, Dr.**
**Liebfrauenstrasse 1 c**
**D-6800 Mannheim 51 (DE)**
Erfinder: **Pommer, Ernst-Heinrich, Dr.**
**Berliner Platz 7**
**D-6703 Limburgerhof (DE)**
Erfinder: **Dressel, Juergen, Dr.**
**Medenheimer Strasse 18**
**D-6708 Neuhofen (DE)**

(56) Entgegenhaltungen:
**CHEMICAL ABSTRACTS, Band 107, Nr. 21, 23. November 1987, Columbus, Ohio, USA; REITER, J.; PONGO, L.; DVORTSAK, P.: "On triazoles. VI. The acylation of 5-amino-1,2,4-triazoles" Seite 751, Spalte 2, Zusammenfassung-Nr. 198186j**

**EP 0 278 445 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung betrifft neue Azolhalbaminal-Derivate und diese enthaltende Mittel zur Nitrifikationsinhibierung von Ammonium-Stickstoff.

Es ist bekannt, daß für Pflanzenernährung geeigneter Stickstoff im Boden in Form von Ammoniumverbindungen und Nitraten vorliegen kann.

Ammoniumstickstoff wird durch Bakterien der Gattungen Nitrosomonas und Nitrobacter über Nitrit- zu Nitratstickstoff (Nitrifikation) oxidiert. Das Ausmaß dieser Prozesse ist im wesentlichen von der Art des Bodens, seinem pH-Wert, seiner Feuchtigkeit und seiner biologischen Aktivität abhängig.

Wegen der kationenfixierenden Eigenschaften des Bodens (Ton, Humus) werden Nitrationen jedoch viel leichter aus dem Boden ausgewaschen als Ammoniumionen. Dies hat zur Folge, daß der ausgewaschene Stickstoff (Nitrationen) einerseits nicht mehr für die Pflanzenernährung zur Verfügung steht und andererseits zu einer unerwünschten Anreicherung von Nitrat im Grundwasser führt. Nitrifikationshemmer (Nitrifikationsinhibitoren) wirken diesen Prozessen entgegen, indem sie die oben angeführte Oxidation der Ammoniumionen inhibieren. Der biologische Mechanismus der Hemmung besteht wahrscheinlich in einer Hemmung des Wachstums der vorgenannten Bakterien.

Heterocyclische Verbindungen, beispielsweise Pyridin-, Pyrimidin-, Thiazol-, Thiadiazol-, Triazol- und Pyrazol-Derivate, sind als nitrifikationsinhibierend bekannt. Solche Verbindungen sind z.B. 2-Chlor-6-trichlormethyl-pyridin, 5-Ethoxi-3-trichlormethyl-1,2,4-thiadiazol und das 3,5-Dimethyl-tetrahydro-1,3,5-thiadiazin-2-thion. Weiter sind bekannt: das 4-Amino-1,2,4-triazol, substituierte Pyrazole (US 3 635 690), N-Acyl-pyrazole (DE—OS 2 745 833, GB 1 592 516, DD 131 063, DD 133 088, US 3 494 757 und SU 1 198 049), Metall-Pyrazol-Komplexe (US 4 523 940) und Pyrazoliumsalz/Dicyandiamid-Gemische (DD 222 471). Ferner sind nitrifikationshemmende 1-Hydroxipyrazol-derivate bekannt (DE 3 409 317).

Die bekannten Wirkstoffe genügen jedoch nicht allen Ansprüchen hinsichtlich Wirksamkeit, Wirkungsdauer, Wirtschaftlichkeit, Unschädlichkeit, anwendungstechnischer Eigenschaften wie Wasserlöslichkeit, Dispergierbarkeit, Dampfdrück usw.. Sie wirken auch sehr unspezifisch und greifen auch Bodenbakterien an, die nicht geschädigt werden sollen.

Die Aufgabe, Nitrifikationsinhibitoren zu finden, deren Eigenschaften auf den genannten Gebieten vorteilhafter sind als die der bisher bekannten Mittel dieser Art, wird gelöst durch Azol-halbaminale der Formel (I)

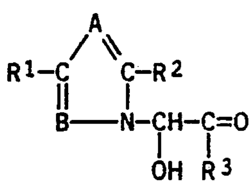

(I)

in der bedeuten:

$R^1$ und $R^2$ unabhängig voneinander Wasserstoff, $C_1$—$C_4$-Alkyl, gegebenenfalls durch Halogen, Alkyl mit 1 bis 4 Kohlenstoffatomen und bis zu 3 gleichen oder unterschiedlichen Halogenatomen substituiertes Aryl oder Halogen,

$R^3$ einen Hydroxirest oder einen offenkettigen oder cyclischen $C_1$—$C_{10}$-alkyl- oder $C_1$—$C_4$-Alkoxi-, $C_2$—$C_4$-Alkenyl-, $C_3$—$C_4$-Alkinyl- oder Styrylrest oder einen Aryl-, Thiophenyl- oder Aralkylrest, wobei der Aromat ein- oder mehrfach durch Halogen, Halogen-$C_1$—$C_4$-alkyl, Halogen-$C_1$—$C_4$-alkoxi, $C_1$—$C_4$-Alkyl, $C_1$—$C_4$-Alkoxi, Carboxi, Carboxi-$C_1$—$C_4$-alkyl, Cyano, Nitro, Sulfoxi oder Sulfonyl substituiert sein kann,

A und B unabhängig voneinander N oder $CR^4$, wobei

$R^4$ Wasserstoff, $C_1$—$C_4$-Alkyl, gegebenenfalls durch Halogen, Alkyl mit 1 bis 4 Kohlenstoffatomen und bis zu 3 gleichen oder unterschiedlichen Halogenatomen substituiertes Aryl oder Halogen bedeutet.

Die Herstellung der neuen Stoffe kann erfolgen durch Umsetzung gegebenenfalls substituierter Azole der Formel (II) oder deren Salzen,

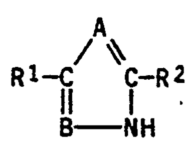

(II)

in welcher $R^1$, $R^2$, A und B die oben angegebene Bedeutung haben mit Glyoxalen, Glyoxylsäure und oder -estern der Formel (III) oder deren Hydraten

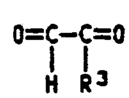

(III)

in welcher $R^3$ die oben angeführte Bedeutung hat.

Die neuen Azol-halbaminale der Formel (I) eignen sich zur Hemmung der Nitrifikation. Überraschend zeigen sie dabei eine bessere und länger anhaltende Wirkung als die bekannten Verbindungen.

$R^1$ bedeutet beispielsweise $C_1$—$C_4$-Alkyl, Methyl, Aryl, Phenyl, Halogen, Chlor, Brom.

$R^2$ bedeutet beispielsweise Wasserstoff.

$R^3$ bedeutet beispielsweise $C_1$—$C_{10}$-Alkyl, Methyl, Ethyl, Propyl, Isopropyl, Butyl, tertiär Butyl, Hexyl, Decyl, $C_1$—$C_4$-Alkoxy, Methoxy, Ethoxy, Butoxy, Aryl, Phenyl, Naphthyl, Styryl, Rest des Thiophens, Halogenphenyl, Chlorphenyl, Dichlorphenyl, $C_1$—$C_4$-Alkoxyphenyl, Methoxyphenyl, Nitrophenyl, $C_6$—$C_8$-Cycloalkyl, Cyclohexyl.

$R^4$ bedeutet beispielsweise Halogen, Chlor, Brom, $C_1$—$C_4$-Alkyl, Methyl.

Die neuen Stoffe sind durch die allgemeine Formel (I) definiert. In dieser Formel stehen:

A und B für N oder $CR^4$,

$R^1$, $R^2$ und $R^4$ unabhängig voneinander vorzugsweise für Halogen, vorzugsweise Chlor oder Brom, Wasserstoff, ein geradkettiges oder verzweigtes Alkyl mit 1 bis 4 Kohlenstoffatomen oder ein gegebenenfalls substituiertes Phenyl, wobei als Substituenten vorzugsweise in Frage kommen: Halogen, Alkyl mit 1 bis 4 Kohlenstoffatomen und biz zu 3 gleiche oder unterschiedliche Halogenatomen, sowie

$R^3$ vorzugsweise für ein geradkettiges oder verzweigtes Alkyl mit 1 bis 10 Kohlenstoffatomen, ein cyclisches Alkyl mit 3 bis 8 Kohlenstoffatomen, ein geradkettiges oder verzweigtes $C_2$—$C_4$-Alken oder $C_3$—$C_4$-Alkin, ein geradkettiger oder verzweigter $C_1$—$C_4$-Alkoxirest oder eine Hydroxigruppe, ein gegebenenfalls substituiertes Phenyl, dessen bevorzugte Substituenten Halogen, insbesondere Chlor, Alkyl mit 1 bis 4 Kohlenstoffatomen, Nitro, oder $C_1$—$C_4$-Alkoxi sind.

Ganz besonders bevorzugt sind solche Verbindungen der Formel (I), in denen A und B unabhängig voneinander N oder $CR^4$, für $R^1$, $R^2$ und $R^4$ unabhängig voneinander Chlor, Brom, Methyl, Ethyl oder Phenyl und für $R^3$ Methyl, Ethyl, n-Propyl, i-Propyl, n-Butyl, s-Butyl, t-Butyl, i-Butyl, Cyclopropyl, Cyclohexyl, Phenyl oder Styryl steht.

Während die Herstellung von Azolhalbaminalen aus Azolen und einfachen Aldehyden sowie deren Verwendung als Synthese-Bausteine bekannt ist (EP 51 278, DE 2 835 158, EP 8 056) sind die Stoffe der Formel (I) neu.

Im allgemeinen lassen sich die neuen Stoffe durch die Umsetzung des Azols mit dem Glyoxalderivat in stöchiometrischen Mengen erhalten. Dabei kann das Azol sowohl als freie Base als auch in Form seines Salzes eingesetzt werden.

Die Glyoxalkomponente kann ebenfalls als reine Substanz oder in Form ihres Hydrates verwendet werden. Es kann jedoch günstig sein das Hydrat vor der Reaktion durch Azeotrop-Destillation zu entwässern.

Die Reaktionen werden allgemein bei −20°C bis 100°C, insbesondere bei 20—40°C durchgeführt. Als Lösungsmittel eignen sich Wasser oder organische Lösungsmittel wie z.B. aliphatische oder aromatische gegebenenfalls chlorierte Kohlenwasserstoffe, Ether, Ketone, Amide, Nitrile und Alkohole.

Falls notwendig, kann die Umsetzung durch Brönstet- oder Lewissäuren, z.B. durch saure Ionenaustauscher, katalysiert werden.

Die in Tabelle 1 aufgeführten neuen Stoffe wurden entsprechend den angegebenen Beispielen hergestellt. Ihre Struktur wurde mit den üblichen Mitteln bestimmt.

## Beispiel 1

3.3 g (29 mMol) frisch destilliertes tert.-Butylglyoxal [vgl. R. C. Fuson, H. Gray, J. J. Gouza; J. Am. Chem. Soc. 61, 1937 (1939)] legte man in 50 ml Methylenchlorid vor. Nachdem man 3 g (29 mMol) 4-Chlorpyrazol hinzugegeben hatte, rührte man 15 h bei Raumtemperatur (20°C), dampfte das Lösungsmittel im Vakuum ab und kristallisierte aus Petrolether um. Man erhielt auf diese Weise 5.0 g (80%) einer weißen Substanz (Verbindung 03 in Tab. 1) mit einem Schmelzpunkt von 104°C.

## Beispiel 2

1.0 g (8.1 mMol) tert.-Butylglyoxal-Hemihydrat wurden mit 50 ml Toluol solange am Rückfluß gekocht, bis sich kein Wasser mehr abschied. Dann werden zu der erkalteten Lösung 0.8 g (8.1 mMol) 4-Chlorpyrazol gegeben und 5 h bei Raumtemperatur gerührt. Nach dem Abziehen des Lösungsmittels im Vakuum verblieben 1,69 g (98%), die bei 55—70°C/l mbar sublimiert werden konnten (Verbindung 03 in Tab. 1, Fp. 104°C).

## Beispiel 3

Zu 6,0 g (25 mMol) einer 30 %igen (Gew.%) wäßrigen Lösung von Methylglyoxal fügte man 1.7 g (25 mMol) Pyrazol und rührte 16 Stunden bei Raumtemperatur. Anschließend extrahierte man 3mal mit Ether, wusch die vereinigten Etherphasen mit gesättigter NaCl-Lösung, trocknete über $MgSO_4$ und dampfte das Lösungsmittel im Vakuum ab. Die Destillation des Rückstandes (70—80°C/l mbar) ergab 2.6 g (74%) einer farblosen, zähflüssigen Substanz (Verbindung 19 in Tab. 1), deren spektroskopische Daten mit der erwarteten Struktur im Einklang stehen.

## Beispiel 4

3.85 g (25 mMol) Phenylglyoxal-Hydrat wurden mit 50 ml Toluol solange am Rückfluß erhitzt bis sich

kein Wasser mehr abschied. Nach dem Erkalten gab man 2.05 g (25 mMol) 3-Methylpyrazol hinzu und rührte ca. 10 min. Im Anschluß konnten 4.5 g (83%) einer ausgefallenen Festsubstanz (Verbindung 22 in Tab. 1) abgesaugt und aus Essigester/Petrolether umkristallisiert werden (Fp. 133°C).

Beispiel 5

In 30 ml Methylenchlorid rührte man 2.0 g (20 mMol) Glyoxylsäureethylester und 2.0 g (20 mMol) 4-Chlorpyrazol 3 Tage bei Raumtemperatur. Nachdem das Ausgangsprodukt dünnschichtchromatographisch nicht mehr nachzuweisen war, wurde das Lösungsmittel im Vakuum abgezogen und das Produkt umkristallisiert. Man isolierte so 4.0 g (100%) einer weißen Substanz (Verbindung 17 in Tab. 1) mit einem Schmelzpunkt von 100°C.

Beispiel 6

Zu 8.1 g (61 mMol) frisch destilliertem tert.-Butylglyoxal in 2.5 ml Benzol tropfte man bei Raumtemperatur 4.0 g (57 mMol) 1,2,4-Triazol in 50 ml Aceton. Nach 16stündigem Nachrühren bei Raumtemperatur wurde der weiße Niederschlag abgesaugt, die Mutterlauge eingeengt und beide Feststoff-Fraktionen gemeinsam aus Petrolether/Ethylacetat umkristallisiert. Man erhielt so 9.1 g (86%) eines Feststoffes, der bei 130°C schmolz (Verbindung 26 in Tab. 1).

Beispiel 7

3.04 g (20 mMol) Phenylglyoxal-Hydrat löste man in 50 ml Toluol und entfernte das enthaltene Wasser durch azeotrope Destillation. Nach dem Erkalten fügte man unter Eiskühlung 2.31 g (20 mMol) 5-Chlor-4-methylimidazol in 30 ml Methylenchlorid hinzu. Anschließend versetzte man mit eine Spatelspitze Ionenaustauscher (H+) und rührte 16 h bei Raumtemperatur. Das ausgefallene weiße Produkt und das Festprodukt, das man nach dem Abziehen des Lösungsmittel im Vakuum erhielt, kristallisierte man aus Petrolether/Ethylacetat um. Man isolierte 4.2 g (84%) einer weißen Substanz (Verbindung 29 in Tab. 1) mit einem Schmelzpunkt von 125°C.

Tabelle 1

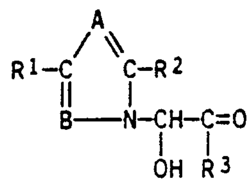

| Nr. | A | B | $R^1$ | $R^2$ | $R^3$ | Fp. [°C] |
|---|---|---|---|---|---|---|
| 01 | C-Cl | N | H | H | $CH_3$ | 81 |
| 02 | C-Cl | N | H | H | $CH(CH_3)_2$ | 103 |
| 03 | C-Cl | N | H | H | $C(CH_3)_3$ | 104 |
| 04 | C-Cl | N | H | H | $C_6H_{13}$ | 118 |
| 05 | C-Cl | N | H | H | $C_{10}H_{21}$ (n-Decyl-) | |
| 06 | C-Cl | N | H | H | $C_6H_{11}$ (Cyclohexyl-) | 124 |
| 07 | C-Cl | N | H | H | $C_6H_5$ (Phenyl) | 178 |
| 08 | C-Cl | N | H | H | 2-$C_{10}H_7$ (2-Naphthyl-) | 200 |
| 09 | C-Cl | N | H | H | (p-Cl)-$C_6H_4$ | 150 |
| 10 | C-Cl | N | H | H | (o-$OCH_3$)-$C_6H_4$ | 74 |
| 11 | C-Cl | N | H | H | (p-$OCH_3$)-$C_6H_4$ | 135 |
| 12 | C-Cl | N | H | H | (p-$NO_2$)-$C_6H_4$ | 110 |
| 13 | C-Cl | N | H | H | (o,p-$Cl_2$)-$C_6H_3$ | 138 |
| 14 | C-Cl | N | H | H | $CH=CH-C_6H_5$ (Styryl-) | 155 |
| 15 | C-Cl | N | H | H | 2-$C_4H_3S$ (2-Thiophenyl) | 171 |
| 16 | C-Cl | N | H | H | OH | 166 |
| 17 | C-Cl | N | H | H | $OC_2H_5$ | 100 |
| 18 | C-Cl | N | H | H | $OC_4H_9$ | 67 |
| 19 | C-H | N | H | H | $CH_3$ | Öl |
| 20 | C-H | N | H | H | $C_6H_5$ | 140 |
| 21 | C-H | N | $CH_3$ | H | $CH_3$ | Öl |
| 22 | C-H | N | $CH_3$ | H | $C_6H_5$ | 133 |
| 23 | C-Br | N | H | H | $C_6H_5$ | 172 |
| 24a | C-Br | N | Br | H | $C_6H_5$ | 74 |
| 24b | C-Br | N | H | Br | $C_6H_5$ | |
| 25 | C-J | N | H | H | $C_6H_5$ | 164 |
| 26 | N | N | H | H | $C(CH_3)_3$ | 130 |
| 27 | N | N | H | H | $C_6H_5$ | 146 |
| 28 | N | C-H | H | H | $C_6H_5$ | 108 |
| 29 | N | C-Cl | $CH_3$ | H | $C_6H_5$ | 125 |
| 30 | N | C-Cl | Cl | H | $C_6H_5$ | 113 |

Ein Trägerstoff ist beispielsweise Wasser, eine organische Flüssigkeit oder ein üblicher inerter fester Trägerstoff z.B. Ton, Quarzmehl, Talkum, Kreide.

Die Wirkung der neuen Verbindungen kann beispielsweise in folgender Weise geprüft werden.

Zu 200 g eines nicht sterilisierten, dem Freiland entnommenen lehmigen Sandbodens, dessen Feuchtigkeitsgehalt auf 50% der maximalen Wasserkapazität eingestellt war, wurden 220 mg Ammoniumsulfat gegeben und mit dem Boden gründlich vermischt. Danach wurden die Wirkstoffe, in 0.2 ml Aceton gelöst, in Mengen von jeweils 1 ppm, bezogen auf feuchten Sandboden, zugesetzt. Nach sorgfältiger Durchmischung wurden die Bodenproben nach Verdunsten des Acetons in mit Aluminiumfolie zur Vermeidung von Wasserverlusten bedeckten 1-Liter-Gläsern zusammen mit den Kontrollen ohne Wirkstoffzusatz über einen Zeitraum von 28 und 42 Tagen bei 21°C bebrütet (Nach diesem Zeitraum enthält eine Bodenprobe mit normaler Bodengare im allgemeinen keine nachweisbaren Mengen von Ammoniumstickstoff mehr).

Danach wurden jeweils 2.5 g der Bodenproben in 100-ml-Erlenmeyerkolben eingefüllt und 22.5 ml einer 0.1n Kaliumsulfat-Lösung zugesetzt. Nach 30minütigem Schütteln wurde abfiltriert und jeweils 2.5 ml der Bodenauszüge wurden mit 1625 ml destilliertem Wasser vermischt. Anschließend wurden zum Nachweis der im Bodenauszug noch vorhandenen Ammoniumionen 1.25 ml Neßler-Reagenz hinzugefügt und gründlich geschüttelt. Die Farbveränderungen wurden dann photometrisch bei einer Wellenlänge von 420 mm gemessen. Anhand von Standardkurven, die durch Messung von Lösungen mit bekannten Ammoniumsulfat-Gehalten ermittelt worden waren, wurden die noch in den Bodenproben vorhandenen Ammoniumsulfat-Mengen bestimmt. Die prozentuale Hemmung der Nitrifikation in den behandelten Bodenproben wurde im Vergleich mit den unbehandelten Bodenproben (nur Ammoniumsulfat-Zusatz) nach folgender Formel berechnet:

$$\ldots\% \text{ Hemmung der Nitrifikation} = \frac{a-b}{a} \times 100$$

a = Nitrifikationsrate von Ammoniumsulfat in Abwesenheit eines Nitrifikationshemmers (mit 100% bzw. 1.0 angenommen)
b = Nitrifikationsrate von Ammoniumsulfat in Gegenwart eines Nitrifikationshemmers

| Wirkstoff Nummer | % Hemmung der Nitrifikation 4 und 6 Wochen nach Zugabe von 1 ppm Wirkstoff im Boden | |
|---|---|---|
| | 4 | 6 |
| 2 | 100 | 95 |
| 3 | 100 | 92 |
| 6 | 100 | 97 |
| 17 | 100 | 89 |
| 18 | 100 | 97 |
| 23 | 100 | 89 |

**Patentansprüche**

1. Azol-halbaminal-derivate der Formel

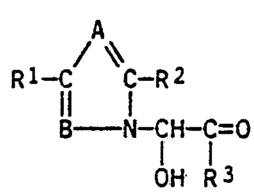

in der bedeuten:

$R^1$ und $R^2$ unabhängig voneinander Wasserstoff, $C_1$—$C_4$-Alkyl, gegebenenfalls durch Halogen, Alkyl mit 1 bis 4 Kohlenstoffatomen und bis zu 3 gleichen oder unterschiedlichen Halogenatomen substituiertes Aryl oder Halogen,

$R^3$ einen Hydroxirest oder einen offenkettigen oder cyclischen $C_1$—$C_{10}$-Alkyl- oder $C_1$—$C_4$-Alkoxi-, $C_2$—$C_4$-Alkenyl-, $C_3$—$C_4$-Alkinyl- oder Styrylrest oder einen Aryl-, Thiophenyl- oder Aralkylrest, wobei der

Aromat ein- oder mehrfach durch Halogen, Halogen-$C_1$—$C_4$-alkyl, Halogen-$C_1$—$C_4$-alkoxi, $C_1$—$C_4$-Alkyl, $C_1$—$C_4$-Alkoxi, Carboxi, Carboxi-$C_1$—$C_4$-alkyl, Cyano, Nitro, Sulfoxi oder Sulfonyl substituiert sein kann,

A und B unabhängig voneinander N oder $CR^4$, wobei

$R^4$ Wasserstoff, $C_1$—$C_4$-Alkyl, gegebenenfalls durch Halogen, Alkyl mit 1 bis 4 Kohlenstoffatomen und bis zu 3 gleichen oder unterschiedlichen Halogenatomen substituiertes Aryl oder Halogen bedeutet.

2. Nitrifikationsinhibierende Mittel, enthaltend einen Trägerstoff und ein Azol-halbaminal-derivat der Formel (I)

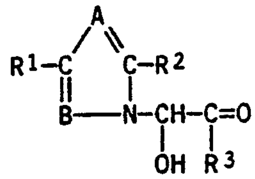

(I)

in der bedeuten

$R^1$ und $R^2$ unabhängig voneinander Wasserstoff, $C_1$—$C_4$-Alkyl, gegebenenfalls durch Halogen, Alkyl mit 1 bis 4 Kohlenstoffatomen und bis zu 3 gleichen oder unterschiedlichen Halogenatomen substituiertes Aryl oder Halogen,

$R^3$ einen Hydroxirest oder einen offenkettigen oder cyclischen $C_1$—$C_{10}$-Alkyl- oder $C_1$—$C_4$-Alkoxi, $C_2$—$C_4$-Alkenyl-, $C_3$—$C_4$-Alkinyl- oder Styrylrest oder einen Aryl-, Thiophenyl- oder Aralkylrest, wobei der Aromat ein- oder mehrfach durch Halogen, Halogen-$C_1$—$C_4$-alkyl, Halogen-$C_1$—$C_4$-alkoxi, $C_1$—$C_4$-Alkyl, $C_1$—$C_4$-Alkoxi, Carboxi, Carboxi-$C_1$—$C_4$-alkyl, Cyano, Nitro, Sulfoxi oder Sulfonyl substituiert sein kann,

A und B unabhängig voneinander N oder $CR^4$, wobei

$R^4$ Wasserstoff, $C_1$—$C_4$-Alkyl, gegebenenfalls durch Halogen, Alkyl mit 1 bis 4 Kohlenstoffatomen und bis zu 3 gleichen oder unterschiedlichen Halogenatomen substituiertes Aryl oder Halogen bedeutet.

3. Verfahren zur Inhibierung der Nitrifikation im Boden, dadurch gekennzeichnet, daß man auf den Boden eine zur Inhibierung ausreichende Menge eines Azol-halbaminalderivats der Formel (I) aufbringt

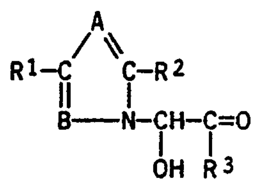

(I)

in der bedeuten

$R^1$ und $R^2$ unabhängig voneinander Wasserstoff $C_1$—$C_4$-Alkyl, gegebenenfalls durch Halogen, Alkyl mit 1 bis 4 Kohlenstoffatomen und bis zu 3 gleichen oder unterschiedlichen Halogenatomen substituiertes Aryl oder Halogen,

$R^3$ einen Hydroxirest oder einen offenkettigen oder cyclischen $C_1$—$C_{10}$-Alkyl- oder $C_1$—$C_4$-Alkoxi-, $C_2$—$C_4$-Alkenyl-, $C_3$—$C_4$-Alkinyl- oder Styrylrest oder einen Aryl-, Thiophenyl- oder Aralkylrest, wobei der Aromat ein- oder mehrfach durch Halogen, Halogen-$C_1$—$C_4$-alkyl, Halogen-$C_1$—$C_4$-alkoxi, $C_1$—$C_4$-Alkyl, $C_1$—$C_4$-Alkoxi, Carboxi, Carboxi-$C_1$—$C_4$-alkyl, Cyano, Nitro, Sulfoxi oder Sulfonyl substituiert sein kann,

A und B unabhängig voneinander N oder $CR^4$, wobei

$R^4$ Wasserstoff, $C_1$—$C_4$-Alkyl, gegebenenfalls durch Halogen, Alkyl mit 1 bis 4 Kohlenstoffatomen und bis zu 3 gleichen oder unterschiedlichen Halogenatomen substituiertes Aryl oder Halogen bedeutet.

4. Verbindung gemäß Anspruch 1, dadurch gekennzeichnet, daß A C—Cl, B N, $R^1$, H, $R^2$H und $R^3$ $C_1$—$C_4$-Alkyl oder Phenyl bedeuten.

5. Verbindung gemäß Anspruch 1, dadurch gekennzeichnet, daß A C—Cl, B N, $R^1$ H, $R^2$ H und $R^3$ $C(CH_3)_3$ bedeuten.

**Revendications**

1. Dérivés d'hémi-aminals d'azole de la formule

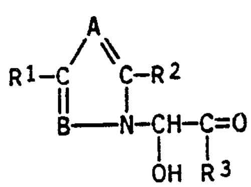

dans laquelle

$R^1$ et $R^2$ représentent, indépendamment l'un de l'autre, hydrogène, alkyle en $C_1$—$C_4$, aryle éventuellement substitué par halogène, alkyle de 1 à 4 atomes de carbone et jusqu'à 3 atomes d'halogène identiques ou différents ou halogène

$R^3$ représente un reste hydroxy ou un reste alkyle en $C_1$—$C_{10}$ ou alcoxy en $C_1$—$C_4$, alcényle en $C_2$—$C_4$, alcynyle en $C_3$—$C_4$ ou styryle, à chaîne ouverte ou cyclique ou un reste aryle, thiophényle ou aralkyle, l'aromate pouvant être substitué une ou plusieurs fois par halogène, halogèn-alkyle en $C_1$—$C_4$, halogèn-alcoxy en $C_1$—$C_4$, alkyle en $C_1$—$C_4$, alcoxy en $C_1$—$C_4$, carboxy, carboxy-alkyle en $C_1$—$C_4$, cyano, nitro, sulfoxy ou sulfonyle

A et B représentent, indépendamment l'un de l'autre, N ou $CR^4$,

$R^4$ représentant hydrogène, alkyle en $C_1$—$C_4$, aryle éventuellement substitué par halogène, alkyle en $C_1$—$C_4$ et jusqu'à 3 atomes d'halogène identiques ou différents.

2. Agent inhibiteur de nitrification, contenant unsupport et un dérivé d'hemi-aminal d'azole de la formule

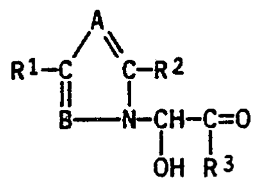

(I)

dans laquelle

$R^1$ et $R^2$ représentent, indépendamment l'un de l'autre, hydrogène, alkyle en $C_1$—$C_4$, aryle éventuellement substitué par halogène, alkyle de 1 à 4 atomes de carbone et jusqu'à 3 atomes d'halogène identiques ou différents ou halogène

$R^3$ représente un reste hydroxy ou un reste alkyle en $C_1$—$C_{10}$ ou alcoxy en $C_1$—$C_4$, alcényle en $C_2$—$C_4$, alcynyle en $C_3$—$C_4$ ou styryle, à chaîne ouverte ou cyclique ou un reste aryle, thiophényle ou aralkyle, l'aromate pouvant être substitué une ou plusieurs fois par halogène, halogèn-alkyle en $C_1$—$C_4$, halogèn-alcoxy en $C_1$—$C_4$, alkyle en $C_1$—$C_4$, alcoxy en $C_1$—$C_4$, carboxy, carboxy-alkyle en $C_1$—$C_4$, cyano, nitro, sulfoxy ou sulfonyle

A et B représentent, indépendamment l'un de l'autre, N ou $CR^4$,

$R^4$ représentant hydrogène, alkyle en $C_1$—$C_4$, aryle éventuellement substitué par halogène, alkyle en $C_1$—$C_4$ et jusqu'à 3 atomes d'halogène identiques ou différents.

3. Procédé d'inhibition de la nitrification dans les sols caractérisé par le fait qu'on applique sur le sol une quantité suffisante pour l'inhibition d'un dérivé d'hémi-aminal d'azole de la formule

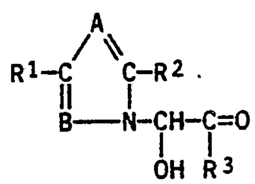

(I)

dans laquelle

$R^1$ et $R^2$ représentent, indépendamment l'un de l'autre, hydrogène, alkyle en $C_1$—$C_4$, aryle éventuellement substitué par halogène, alkyle de 1 à 4 atomes de carbone et jusqu'à 3 atomes d'halogène identiques ou différents ou halogène

$R^3$ représente un reste hydroxy ou un reste alkyle en $C_1$—$C_{10}$ ou alcoxy en $C_1$—$C_4$, alcényle en $C_2$—$C_4$, alcynyle en $C_3$—$C_4$ ou styryle, à chaîne ouverte ou cyclique ou un reste aryle, thiophényle ou aralkyle, l'aromate pouvant être substitué une ou plusieurs fois par halogène, halogèn-alkyle en $C_1$—$C_4$, halogèn-alcoxy en $C_1$—$C_4$, alkyle en $C_1$—$C_4$, alcoxy en $C_1$—$C_4$, carboxy, carboxy-alkyle en $C_1$—$C_4$, cyano, nitro, sulfoxy ou sulfonyle

A et B représentent, indépendamment l'un de l'autre, N ou $CR^4$,

$R^4$ représentant hydrogène, alkyle en $C_1$—$C_4$, aryle éventuellement substitué par halogène, alkyle en $C_1$—$C_4$ et jusqu'à 3 atomes d'halogène identiques ou différents.

4. Composé selon la revendication 1, caractérisé par le fait qu'A représente C—Cl, B représente N, $R^1$, H, $R^2$, H et $R^3$, alkyle en $C_1$—$C_4$ ou phényle.

5. Composé selon la revendication 1, caractérisé par le fait que A représente C—Cl, B représente N, $R^1$, H, $R^2$, H et $R^3$, $C(CH_3)_3$.

## Claims

1. An azole hemiaminal derivative of the formula

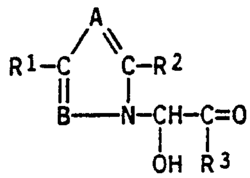

where $R^1$ and $R^2$ independently of one another are each hydrogen, $C_1$—$C_4$-alkyl, aryl which may be substituted by halogen, alkyl of 1 to 4 carbon atoms and by up to 3 identical or different halogen atoms, or halogen,

$R^3$ is hydroxyl or an open-chain or cyclic $C_1$—$C_{10}$-alkyl or $C_1$—$C_4$-alkoxy, $C_2$—$C_4$-alkenyl, $C_3$- or $C_4$-alkynyl or styryl radical or aryl, thiophenyl or aralkyl, where the aromatic radical may be monosubstituted or polysubstituted by halogen, halo-$C_1$—$C_4$-alkyl, halo-$C_1$—$C_4$-alkoxy, $C_1$—$C_4$-alkyl, $C_1$—$C_4$-alkoxy, carboxyl, carboxy-$C_1$—$C_4$-alkyl, cyano, nitro, sulfoxyl or sulfonyl, A and B independently of one another are each N or $CR^4$, and $R^4$ is hydrogen, $C_1$—$C_4$-alkyl, aryl which may be substituted by halogen, alkyl of 1 to 4 carbon atoms and by up to 3 identical or different halogen atoms, or halogen.

2. A nitrification inhibitor containing a carrier and an azole hemiaminal derivative of the formula (I)

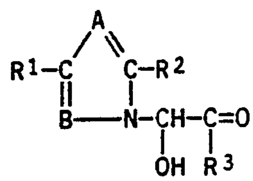

(I)

where $R^1$ and $R^2$ independently of one another are each hydrogen, $C_1$—$C_4$-alkyl, aryl which may be substituted by halogen, alkyl of 1 to 4 carbon atoms and by up to 3 identical or different halogen atoms, or halogen,

$R^3$ is hydroxyl or an open-chain or cyclic $C_1$—$C_{10}$-alkyl or $C_1$—$C_4$-alkoxy, $C_2$—$C_4$-alkenyl, $C_3$- or $C_4$-alkynyl or styryl radical or aryl, thiophenyl or aralkyl, where the aromatic radical may be monosubstituted or polysubstituted by halogen, halo-$C_1$—$C_4$-alkyl, halo-$C_1$—$C_4$-alkoxy, $C_1$—$C_4$-alkyl, $C_1$—$C_4$-alkoxy, carboxyl, carboxy-$C_1$—$C_4$-alkyl, cyano, nitro, sulfoxyl or sulfonyl, A and B independently of one another are each N or $CR^4$, and $R^4$ is hydrogen, $C_1$—$C_4$-alkyl, aryl which may be substituted by halogen, alkyl of 1 to 4 carbon atoms and by up to 3 identical or different halogen atoms, or halogen.

3. A process for inhibiting nitrification in the soil, wherein an azole hemiaminal derivative of the formula (I)

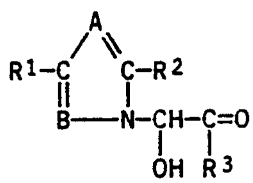

(I)

where $R^1$ and $R^2$ independently of one another are each hydrogen, $C_1$—$C_4$-alkyl, aryl which may be substituted by halogen, alkyl of 1 to 4 carbon atoms and by up to 3 identical or different halogen atoms, or halogen,

$R^3$ is hydroxyl or an open-chain or cyclic $C_1$—$C_{10}$-alkyl or $C_1$—$C_4$-alkoxy, $C_2$—$C_4$-alkenyl, $C_3$- or $C_4$-alkynyl or styryl radical or aryl, thiophenyl or aralkyl, where the aromatic radical may be monosubstituted or polysubstituted by halogen, halo-$C_1$—$C_4$-alkyl, halo-$C_1$—$C_4$-alkoxy, $C_1$—$C_4$-alkyl, $C_1$—$C_4$-alkoxy, carboxyl, carboxy-$C_1$—$C_4$-alkyl, cyano, nitro, sulfoxyl or sulfonyl, A and B independently of one another are each N or $CR^4$, and $R^4$ is hydrogen, $C_1$—$C_4$-alkyl, aryl which may be substituted by halogen, alkyl of 1 to 4 carbon atoms and by up to 3 identical or different halogen atoms, or halogen, is applied to the soil in an amount sufficient for inhibition.

4. A compound as claimed in claim 1, wherein A is C—Cl, B is N, $R^1$ and $R^2$ are each H and $R^3$ is $C_1$—$C_4$-alkyl or phenyl.

5. A compound as claimed in claim 1, wherein A is C—Cl, B is N, $R^1$ and $R^2$ are each H and $R^3$ is $C(CH_3)_3$.